# FASCICULE DE BREVET EUROPEEN

(11) **EP 4 340 630 B1**
(45) Date de publication et mention de la délivrance du brevet: **27.08.2025**
(21) Numéro de dépôt: 22729210.9
(22) Date de dépôt: 17.05.2022
(51) Int. Cl.: A23J 1/14, A23L 33/18

(54) **EXTRAIT DE VACCINIUM MACROCARPON ET SES EFFETS COSMÉTIQUES ANTI-ÂGE**
VACCINIUM-MAKROCARPON-EXTRAKT UND KOSMETISCHE ANTI-AGING-WIRKUNG DAVON
VACCINIUM MACROCARPON EXTRACT AND ANTI-AGEING COSMETIC EFFECTS THEREOF

(30) Priorité: 17.05.2021 FR 2105098
(43) Date de publication de la demande: 27.03.2024
(73) Titulaire: Societe Industrielle Limousine d'Application Biologique, 19240 Saint Viance (FR)
(72) Inventeur: PAUFIQUE, Jean, 19130 OBJAT (FR)
(74) Mandataire: Aquinov
(86) Numéro de dépôt international: PCT/EP2022/063237
(87) Numéro de publication internationale: WO 2022/243268

(56) Documents cités:
- EP-A1- 1 661 576
- DE-A1- 102008 012 068
- FR-A1- 3 016 521
- US-A1- 2004 191 335
- SPADONI ANDREANI EUGENIO ET AL: "Comparison of enzymatic and microwave-assisted alkaline extraction approaches for the generation of oligosaccharides from American Cranberry ( Vaccinium macrocarpon ) Pomace", vol. 85, no. 8, 1 August 2020 (2020-08-01), US, pages 2443 - 2451, XP055875767, ISSN: 0022-1147, Retrieved from the Internet <URL:https://onlinelibrary.wiley.com/doi/full-xml/10.1111/1750-3841.15352> DOI: 10.1111/1750-3841.15352

## Description

### Domaine technique

L'invention concerne un extrait de *Vaccinium macrocarpon* comprenant au moins 80% de peptides en poids de matières sèches de l'extrait, en particulier un principe actif cosmétique le comprenant, une composition cosmétique comprenant ledit principe actif et ses utilisations cosmétiques anti-âge.

### Etat de l'art

L'essor des visioconférences a profondément fait évoluer nos modes de communication personnel et professionnel et a créé de nouvelles préoccupations cosmétiques, à l'instar de la *Zoom face.* Ce phénomène intervient lorsque notre visage apparaît en direct et en gros plan sur l'écran et que l'on ne peut s'empêcher d'analyser les rides et autres défauts s'animant en même temps que nos expressions. Ainsi, le boom des réunions virtuelles a entraîné celui des recherches internet sur les solutions anti-âge globales.

L'offre des ingrédients anti-âge est pléthorique. De nombreuses molécules actives sont déjà connues pour leur efficacité cosmétique et leur innocuité. Parmi ces molécules dites de référence, on peut citer le rétinol, la niacinamide, les vitamines C et E ou encore les peptides. Ces derniers s'imposent comme des ingrédients incontournables et ce depuis les années 1980, période durant laquelle ils ont commencé à être intégrés dans des produits cosmétiques.

La plupart des peptides présents sur le marché sont d'origine synthétique, issus de l'industrie de la chimie ou des biotechnologies. Les peptides synthétiques sont dotés d'une efficacité certes puissante mais présentant l'inconvénient d'être restreinte à une cible biologique. Bien qu'ils soient décrits comme naturels de par leur composition en acides aminés, leur production par la chimie nécessite l'emploi de solvants toxiques. La synthèse par les biotechnologies, quant à elle, nécessite d'avoir recours à des organismes génétiquement modifiés (OGM). Ces modes de fabrication ne sont plus en phase avec les besoins actuels des consommateurs qui placent les soins cosmétiques issus du monde botanique en tête de leurs attentes. Le document DE102008012068 décrit l'utilisation en cosmétique d'un extrait de Cranberry(Vaccinium macrocarpon) dans le domaine capillaire. La fraction peptide est obtenue par un procédé d'hydrolyse enzymatique qui va résulter dans l'obtention d'un extrait comprenant des oligosaccharides et des peptides.

Or, le monde végétal recèle une grande diversité de molécules parmi lesquelles les peptides naturels s'illustrent comme des composés actifs très prometteurs. Bien que certains peptides existent librement sous cette forme, la plupart sont issus de protéines natives dont ils peuvent être libérés par hydrolyse.

Il existe donc un besoin pour de nouveaux ingrédients cosmétiques d'origine naturelle et constitués par des peptides d'origine naturelle pour lutter contre le vieillissement cutané et présentant ainsi une efficacité anti-âge globale.

### Résumé de l'invention

Pour répondre à ce besoin, l'invention propose un nouvel extrait obtenu à partir de la canneberge (*Vaccinium macrocarpon*) comprenant au moins 80% de peptides en poids de matières sèches de l'extrait, en particulier il s'agit d'un principe actif cosmétique comprenant ledit extrait pour une application cosmétique anti-âge.

La canneberge (*Vaccinium macrocarpon* (A.)) est un arbrisseau à feuilles persistantes mesurant une trentaine de centimètres. Elle pousse à l'état sauvage dans les tourbières acides des régions froides, les forêts montagneuses et les prés sablonneux d'Amérique du Nord. Quand vient l'été, des fleurs campanulées roses et pourpres offrent leur corolle ouverte aux pollinisateurs. Au fil de la saison, le développement des baies rouges métamorphose ces régions en de vastes étendues flamboyantes. C'est à l'automne que les fruits arrivés à maturité sont récoltés.

La canneberge, aussi nommée Atoca par les amérindiens, était traditionnellement employée pour ses propriétés médicinales. Lors de l'arrivée des premiers européens sur le territoire américain, les colons ont à leur tour découvert et apprécié cette baie au goût acidulé. A partir du milieu du 19^{ème} siècle, des médecins allemands contribuèrent à répandre sa consommation dans le monde moderne. La canneberge devint peu à peu l'aliment santé de notre époque et est considérée de nos jours comme un véritable super-fruit.

La canneberge est notamment bien connue pour son intérêt nutritionnel et ses bénéfices sur la santé, en particulier pour ses effets antioxydants. En effet, la canneberge est riche en vitamine C et en autres antioxydants permettant de prévenir l'apparition de certains cancers, de maladies cardiovasculaires, et de diverses maladies liées au vieillissement. La canneberge est également utilisée dans la prévention des infections urinaires mais également pour soigner ou prévenir la gingivite et la parodontite.

Enfin, des vertus anti-âge ont pu lui être attribuées du fait de la présence de nombreuses molécules anti-oxydantes.

La présente invention se rapporte à un extrait spécifique riche en peptides et dépourvu des molécules anti-oxydantes naturellement présentes dans le fruit, et présentant une efficacité cosmétique, en particulier anti-âge.

Ainsi, l'invention concerne un extrait de *Vaccinium macrocarpon* comprenant au moins 80% de peptides en poids de matières sèches de l'extrait.

Préférentiellement, ledit extrait est obtenu à partir des fruits de *Vaccinium macrocarpon,* plus préférentiellement à partir des tourteaux de *Vaccinium macrocarpon.*

Ledit extrait est un principe actif cosmétique particulièrement d'intérêt comme ingrédient naturel pour lutter contre le vieillissement cutané.

Ainsi, le principe actif cosmétique selon l'invention comprenant les peptides d'origine naturelle présente avantageusement un effet anti-âge globale, en particulier il présente un effet anti-rides et/ou permet d'améliorer l'éclat du teint et/ou d'améliorer la qualité de la jonction dermo-épidermique, et/ou d'améliorer l'homéostasie cutanée.

Il répond donc aux problématiques de l'art antérieur et la présente invention vise les applications cosmétiques citées ci-dessus, préférentiellement lorsque le principe actif est intégré dans une composition cosmétique comprenant au moins 0,1% dudit principe actif et un milieu physiologiquement acceptable, pour une application topique.

La présente invention se rapporte également à un procédé d'obtention du principe actif selon l'invention comprenant notamment une solubilisation des tourteaux de fruits de *Vaccinium macrocarpon* dans l'eau, suivi d'hydrolyses enzymatiques, d'une séparation de la phase soluble et insoluble et de la récupération de la phase soluble, d'une inactivation enzymatique, d'une purification de la fraction peptidique et éventuellement d'une concentration de l'extrait suivi d'une filtration stérilisante.

D'autres caractéristiques et avantages ressortiront de la description détaillée de l'invention et des exemples qui vont suivre.

### Description détaillée de l'invention

### Définition

Par « principe actif cosmétique » au sens de l'invention, on entend un extrait comprenant au moins une molécule, préférentiellement un ensemble de molécules, plus préférentiellement les molécules sont des peptides, présentant un effet cosmétique sur la peau. Préférentiellement l'effet cosmétique sur la peau est un effet anti-âge.

Par « *Vaccinium macrocarpon* » au sens de l'invention, on entend un arbuste de la famille des *Ericacées,* également connu sous le nom de Canneberge à gros fruits, Airelle à gros fruits, Cranberry (en Anglais) ou encore sous les noms scientifiques : *Oxycoca macrocarpa (A.), Oxycoccus macrocarpus (A.)* ou *Schollera macrocarpos (A.).*

Par « hydrolysat de « *Vaccinium macrocarpon* » au sens de l'invention, on entend tout principe actif issu des fruits de *Vaccinium macrocarpon,* en particulier des tourteaux de *Vaccinium macrocarpon,* obtenu par un procédé comprenant au moins une étape d'hydrolyse, préférentiellement deux étapes d'hydrolyses successives de *Vaccinium macrocarpon.* Le terme hydrolysat de *Vaccinium macrocarpon* exclut les molécules extraites uniquement par macération ou décantation de *Vaccinium macrocarpon.*

Par « peptides » au sens de l'invention, on entend des peptides de taille, exprimée en masse molaire, inférieure ou égale à 2 000 Da.

Par « acides aminés acides » au sens de l'invention, on entend un acide aminé chargé négativement à pH neutre, tel que l'acide aspartique ou l'acide glutamique.

Par « acides aminés basiques » au sens de l'invention, on entend un acide aminé chargé positivement à pH neutre, tel que l'arginine, l'histidine ou la lysine.

Par « peptides cationiques » au sens de l'invention, on entend des peptides de taille, exprimée en masse molaire, inférieure ou égale à 2 000 Da et formés majoritairement d'acides aminés basiques.

Par « tourteaux » au sens de l'invention, on entend les résidus solides issus de l'extraction de l'huile des graines ou des fruits, en particulier des graines et de la pulpe de *Vaccinium macrocarpon.* Les graines et la pulpe sont notamment broyées et l'huile est extraite par un procédé d'extraction par pression à froid bien connu de l'homme du métier. Ce procédé permet de récupérer l'huile de canneberge et les co-produits résiduels solides : les tourteaux.

Par « film » au sens de l'invention on entend un produit cosmétique que l'on applique momentanément sur la peau et que l'on retire après un certain temps d'application, présentant un effet cosmétique ou dermocosmétique. Il peut, par exemple, s'agir d'un masque, d'un patch ou d'une bande, pour le visage ou le corps.

### Principe actif selon l'invention

La présente invention a donc pour objet un principe actif cosmétique comprenant au moins un extrait de *Vaccinium macrocarpon* comprenant au moins 80% de peptides en poids de matières sèches de l'extrait.

L'extrait selon l'invention est un ingrédient d'origine naturelle, y compris les peptides présents dans ledit extrait. La spécificité de l'extrait selon l'invention est qu'il est constitué d'au moins 80% de peptides d'origine naturelle c'est-à-dire qu'ils ne sont pas synthétiques et donc issus de l'industrie de la chimie ou des biotechnologies. Pour cela, l'extrait est obtenu à partir de *Vaccinium macrocarpon,* préférentiellement des fruits, plus préférentiellement des tourteaux de *Vaccinium macrocarpon.*

Les tourteaux de *Vaccinium macrocarpon* sont préférentiellement obtenus après une première étape d'extraction des fruits de la canneberge permettant d'une part de récolter le jus de canneberge et d'autre part les graines et la pulpe résiduelle issus du fruit. Les graines et la pulpe sont ensuite broyés et l'huile extraite par pression à froid. Subsiste alors le tourteau de canneberge, matière première de l'extrait selon l'invention.

Selon un mode de réalisation préféré, l'extrait selon l'invention est un hydrolysat des tourteaux de *Vaccinium macrocarpon,* préférentiellement un hydrolysat enzymatique des tourteaux de *Vaccinium macrocarpon.*

De façon particulièrement avantageuse, l'hydrolysat enzymatique est obtenu après deux hydrolyses enzymatiques successives mis en œuvre par deux protéases différentes.

L'extrait selon l'invention comprend alors préférentiellement au moins 80% de peptides en poids de matière sèche de l'extrait. Selon un mode de réalisation particulièrement préféré, les peptides représentent 98% en poids de matière sèche de l'extrait. Lesdits peptides ont une taille, exprimé en masse molaire, inférieure ou égale à 2 000 Da. Préférentiellement, lesdits peptides ont une taille comprise entre 243 Da et 2 000 Da.

La répartition et la quantité des peptides peut être déterminée par un dosage spectro-photométrique selon la méthode de Lowry (Lowry et al., Protein measurement with the Folin reagent, J. Biol. Chem., 193, 265-275, 1951). La caractérisation et la quantification des acides aminés présents dans l'extrait et donc dans le principe actif selon l'invention peuvent être réalisées par chromatographie liquide après hydrolyse totale de l'échantillon.

Ainsi, l'extrait selon l'invention est préférentiellement et majoritairement composé de peptides comprenant entre 4 et 6 acides aminés, lesdits peptides étant enrichis en acides aminés basiques, préférentiellement au moins 20%, plus préférentiellement au moins 25%, encore plus préférentiellement 27,9%, particulièrement en arginine, préférentiellement au moins 15%, plus préférentiellement 19,6%.

Ainsi, l'extrait selon l'invention présente un taux de peptides cationiques, lesdits peptides étant formés majoritairement d'acides aminés basique, supérieur à 20%.

L'inventeur a également déterminé la teneur en matière sèche, la teneur en cendres et la teneur en sucres totaux de l'extrait.

La teneur en matière sèche peut être déterminée par la pesée des résidus issus du séchage des échantillons de l'extrait selon l'invention à 105°C dans une étuve jusqu'à l'obtention d'un poids constant.

La teneur en cendres minérales peut être déterminée par la pesée des résidus issus de l'incinération des échantillons du principe actif selon l'invention à 550°C dans un four à moufle électrique.

La teneur en sucres totaux (carbohydrates) dans le principe actif comprenant ledit extrait peut être déterminée par la méthode de DUBOIS (Dubois M. et al., Analytical chemistry, 28, 3, 350-356, 1956). La teneur en sucres totaux dans le principe actif est exprimée en pourcentage par rapport à la matière sèche.

Le dosage de l'azote total dans l'extrait selon l'invention peut être réalisé notamment par la méthode de KJELDHAL (référence : Official method of analysis of the A.O.C., 12th ed. W Horwitz, E.D., New-York, 15-60, 1975). La teneur est exprimée en pourcentage par rapport à la teneur en matières sèches.

L'extrait selon l'invention comprend préférentiellement 95% de fraction azotée (soit les peptides) et 5% de cendres, en poids par rapport à la matière sèche. Il ne comprend donc pas de sucres.

Le principe actif selon l'invention comprend l'extrait seul ou avec d'autres constituants.

Le principe actif cosmétique selon l'invention peut se présenter sous forme liquide ou sous forme solide ou sous forme de film.

Lorsqu'il se présente sous forme liquide, le principe actif selon l'invention est préférentiellement exclusivement constitué par l'extrait de tourteaux de *Vaccinium macrocarpon* accompagné de stabilisant et/ou de conservateurs.

L'extrait sous forme liquide se présente préférentiellement sous forme d'une solution aqueuse liquide limpide, avec une faible odeur et une couleur jaune très clair. Il peut toutefois être plus coloré.

Préférentiellement, l'extrait selon l'invention sous forme liquide a une teneur en matières sèches de : 5 g/L à 30 g/L, encore plus préférentiellement de 9 g/L à 14 g/L.

Lorsque le principe actif selon l'invention se présente sous forme solide, il est préférentiellement sous forme de poudre avec ou sans support. Le support peut être, par exemple choisi parmi les maltodextrines. Dans ce cas, préférentiellement, l'extrait représente au moins 5% en poids et le support au plus 95% en poids.

Le principe actif selon l'invention peut aussi être présenté sous forme d'un film. Dans ce cas, l'extrait de tourteaux de *Vaccinium macrocarpon* représente préférentiellement au moins 0,1% en poids du film.

Lorsqu'il se présente sous forme de film, le principe actif comprend :
- au moins l'hydrolysat de tourteaux de *Vaccinium macrocarpon* selon l'invention.
- au moins une charge minérale, et
- au moins un polymère d'origine naturelle, et
- au moins un plastifiant, et
- au moins un tensio-actif, et

Le polymère d'origine naturelle peut être choisi parmi la : Pectine, Gomme tamarin, Alginate, Pullulane, Psyllium, Xanthane, Guar, Tara, Caroube, Agar, Gomme arabique, Gellane, Dextran, Carraghénane, Cellulose, Konjac et le Chitosan.

Le plastifiant peut être choisi parmi : Glycérol, Sorbitol, Saccharose, Erythritol, Urée, Propylène glycol et le Butylene glycol.

La charge minérale peut être choisie parmi : Carbonate de calcium, argile verte, kaolin, perlite, talc, silicate de magnésium, mica, sericite diatomée, silice, sulfate de calcium chlorure de calcium, chlorure de potassium, oxyde de fer et l'oxyde de zinc.

Le principe actif peut comprendre en outre un pigment pour colorer le film.

Selon un mode de réalisation préféré, l'extrait du principe actif cosmétique selon l'invention est susceptible d'être obtenu par un procédé d'extraction comprenant au moins une hydrolyse enzymatique de tourteaux de *Vaccinium macrocarpon,* les tourteaux ayant été préalablement solubilisés dans l'eau.

Selon un mode de réalisation particulièrement préféré, l'extrait du principe actif cosmétique selon l'invention est susceptible d'être obtenu par un procédé d'extraction comprenant au moins deux hydrolyses enzymatiques de tourteaux de *Vaccinium macrocarpon,* les tourteaux ayant été préalablement solubilisés dans l'eau.

Préférentiellement les deux hydrolyses enzymatiques, successives, sont réalisées avec deux protéases différentes. Le procédé comprend préférentiellement ensuite un tri des molécules présentes dans la phase soluble sélectionnée après hydrolyse, pour purifier la fraction peptidique et particulièrement les peptides de tailles inférieures à 2000 Da.

Ainsi, l'extrait du principe actif cosmétique selon l'invention est susceptible d'être obtenu par le procédé suivant :
- solubilisation d'au moins 100 g/L de tourteaux de *Vaccinium Macrocarpon* dans l'eau,
- hydrolyse enzymatique,
- purification de la fraction peptidique.

Selon un mode de réalisation particulièrement préféré, l'extrait du principe actif selon l'invention est susceptible d'être obtenu par un procédé tel que décrit ci-après.

### Procédé d'obtention de l'extrait selon l'invention

L'extrait selon l'invention peut être obtenu par tout moyen.

Selon un mode de réalisation particulièrement adapté, l'extrait selon l'invention est obtenu par la mise en œuvre des étapes suivantes :
- Solubilisation des fruits de *Vaccinium macrocarpon* dans l'eau, préférentiellement des tourteaux de *Vaccinium macrocarpon,* préférentiellement à raison d'au moins 100g des tourteaux de *Vaccinium macrocarpon* par litre d'eau,
- au moins deux hydrolyses enzymatiques, successives, préférentiellement au moins deux hydrolyses enzymatiques réalisées à l'aide d'au moins deux protéases différentes,
- séparation des phases soluble et insoluble, par tout moyen permettant de séparer une phase insoluble de la phase soluble, par exemple par centrifugation, filtration ou décantation,
- récupération de la phase soluble,
- inactivation enzymatique par tout moyen permettant l'inactivation des activités enzymatiques, par exemple par traitement thermique,
- tri moléculaire des molécules de la phase soluble pour récupérer les molécules de tailles inférieures à 2000 Da et notamment des peptides de tailles inférieures à 2000 Da, préférentiellement de taille comprise entre 243 Da et 2000 Da, par tout moyen permettant le tri moléculaire, par exemple par ultrafiltration ou chromatographie ionique,
- éventuellement désodorisation ou décoloration,
- préférentiellement filtration et filtration stérilisante.

Lors de l'extraction du jus de canneberge, subsiste les graines et la pulpe des fruits. Dans un second temps, lors de l'extraction de l'huile par pression à froid, seule subsiste les tourteaux. Ce processus est usuel dans le domaine de l'industrie agroalimentaire. Ces co-produits sont ensuite réduits en poudre. Ainsi, seules les molécules des fruits, préférentiellement des tourteaux constituent la matière première de départ.

Le tri moléculaire peut être réalisé par ultrafiltration, préférentiellement le tri moléculaire est réalisé par chromatographie ionique.

Une étape supplémentaire pour sécher l'hydrolysat peut être ajoutée de façon à ce qu'il se présente sous forme solide, préférentiellement sous forme de poudre. Il peut s'agir d'une étape d'atomisation ou de lyophilisation par exemple. Dans ce cas un support comme par exemple une maltodextrine peut être utilisée.

Les étapes des procédés décrits ci-avant, prises individuellement, sont usuelles dans le domaine des extractions d'actifs à partir de matières premières naturelles et l'homme du métier est à même d'en ajuster les paramètres réactionnels sur la base de ses connaissances générales, et de façon à obtenir les caractéristiques du principe actif objet de l'invention.

### Composition cosmétique

Le principe actif selon l'invention, est de préférence utilisé dans des compositions cosmétiques comprenant un milieu physiologiquement acceptable, préférentiellement un milieu cosmétiquement acceptable. Il s'agit de compositions de différentes formes galéniques, adaptées à une application par voie topique sur la peau.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux, poudres, ou fond de teint. Elles peuvent être plus ou moins fluides et avoir l'aspect de crèmes, émulsions, gels, masques ou tous autres aspects des cosmétiques de soin de la peau saine.

Il peut s'agir de compositions comprenant au moins 0,1% du principe actif liquide selon l'invention (soit environ 7,2 ppm de peptides), préférentiellement entre 0,5 et 10% ou comprenant au moins 0,01% d'un principe actif poudre selon l'invention.

Ces compositions comprennent, outre le principe actif, un milieu physiologiquement acceptable et de préférence cosmétiquement acceptable, c'est-à-dire qui ne provoque pas de sensations d'inconfort pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles,
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic Ingredient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont destinées à être utilisées sur des peaux saines, notamment des peaux matures, en particulier pour un effet anti-âge global, préférentiellement un effet anti-rides et/ou pour améliorer l'éclat du teint et/ou améliorer la qualité de la jonction dermo-épidermique, et/ou améliorer l'homéostasie cutanée.

L'invention a donc également pour objet un procédé cosmétique de traitement de la peau pour un effet anti-rides et/ou pour améliorer l'éclat du teint et/ou améliorer la qualité de la jonction dermo-épidermique, et/ou améliorer l'homéostasie cutanée qui consiste en l'application topique sur la peau d'une personne saine d'un tel extrait selon l'invention ou d'une telle composition selon l'invention.

### Utilisation

Le principe actif selon l'invention présente ainsi des caractéristiques qui permettent son utilisation en cosmétique et en particulier pour limiter les effets naturels liés au vieillissement et ainsi obtenir un effet anti-âge global, préférentiellement un effet anti-rides et/ou pour améliorer l'éclat du teint et/ou améliorer la qualité de la jonction dermo-épidermique, et/ou améliorer l'homéostasie cutanée.

Il permet ainsi d'améliorer l'homéostasie de la peau et donc la fonction barrière chez des sujets sains âgés, ce qui se traduit directement par une réduction des pertes insensibles en eau de 15%. Le rayonnement est également augmenté de 14%, ce qui se traduit par un éclat du teint ravivé. La fonction barrière épidermique des peaux âgées est donc renforcée.

L'extrait selon l'invention favorise également l'expression des biomarqueurs structuraux majeurs du derme tels que les collagènes I et III ; la périostine ; la tropoélastine ; les protéoglycanes (décorine, perlécane, byglicane, aggrécane) ; l'acide hyaluronique.

L'extrait selon l'invention permet également de diminuer l'expression des marqueurs de dégradation du derme tels que les MMP-1.

Ainsi, la qualité des fibres du derme est améliorée de 17% ce qui se traduit par une réduction des rides de la patte d'oie chez des volontaires caucasiens et asiatiques.

En agissant sur ces différents marqueurs, le principe actif selon l'invention a une action anti-âge globale. Par conséquent, la beauté et la qualité de la peau sont sublimées, les rides sont significativement réduites, la peau est plus ferme et l'éclat du teint ravivé. Il est particulièrement adapté aux peaux saines matures.

Le principe actif comprenant au moins un extrait des tourteaux de *Vaccinium macrocarpon,* peut ainsi être utilisé pour des utilisations cosmétiques et donc par définition non thérapeutiques, anti-âge en application topique sur la peau saine.

Le principe actif se différencie des extraits de canneberge communément connus pour leur effet anti oxydant puisqu'il ne contient pas de polyphénols lui conférant cette efficacité.

L'invention est à présent illustrée par des exemples non limitatifs de compositions selon l'invention et par des résultats d'essais.

### Exemples

### Exemple 1 : extrait de Vaccinium macrocarpon sans procédé d'extraction selon l'invention (Hors Invention).

La caractérisation et la quantification des acides aminés constitutifs d'un tourteau de Canneberge ont été réalisées par chromatographie liquide.

**[Tableau 1]**

| **Nom de l'acide aminé** | **Répartition (%)** |
|---|---|
| Alanine | 5,0 |
| Arginine | 12,2 |
| Acide aspartique | 10,8 |
| Acide glutamique | 22,3 |
| Cystine | 0,0 |
| Glycine | 5,8 |
| Histidine | 2,2 |
| Isoleucine | 4,3 |
| Leucine | 7,9 |
| Lysine | 2,9 |
| Phénylalanine | 5,0 |
| Proline | 2,9 |
| Serine | 5,0 |
| Thréonine | 3,6 |
| Tyrosine | 3,6 |

Le tourteau de canneberge présente un taux d'acides aminés basiques (arginine, histidine, lysine) de 17,3% et un taux d'acides aminés acides (acide aspartique, acide glutamique) de 33,1%.

### Exemple 2 : Extrait selon l'invention

Le principe actif est obtenu par la mise en œuvre des étapes suivantes :
- solubilisation dans l'eau des tourteaux de *Vaccinium macrocarpon* à raison de 100 g/L,
- hydrolyse enzymatique,
- inactivation enzymatique par traitement thermique,
- une étape de tri moléculaire par chromatographie ionique, destinée à sélectionner les molécules, notamment les peptides, de tailles inférieures à 2000 Da,
- désodorisation,
- filtrations et filtration stérilisante.

Le principe actif obtenu présente les caractéristiques analytiques suivantes :
- un taux de matière sèche : 12,2 g/L
- une teneur en protéine : 10,2g/L (83,6%)
- un pH : 3,0 - 4,0
- un taux de cendres : 0,6g/L (4,9%).

La caractérisation et la quantification des acides aminés constitutifs de l'extrait selon l'invention ont été réalisées par chromatographie liquide après hydrolyse de l'échantillon.

La répartition des différents acides aminés est présentée dans le tableau 2

**[Tableau 2]**

| **Nom de l'acide aminé** | **Répartition (%)** |
|---|---|
| Alanine | 6,2 |
| Arginine | 19,6 |
| Acide aspartique | 10,3 |
| Acide glutamique | 20,6 |
| Cystine | 0,0 |
| Glycine | 5,2 |
| Histidine | 3,1 |
| Isoleucine | 4,1 |
| Leucine | 8,2 |
| Lysine | 5,2 |
| Phénylalanine | 2,1 |
| Proline | 0,0 |
| Serine | 5,2 |
| Thréonine | 3,1 |
| Tyrosine | 3,1 |

L'extrait selon l'invention possède un taux d'acides aminés basiques supérieur (27,9%) par rapport au tourteau de canneberge de l'exemple 1 en raison d'une teneur en arginine importante et d'un taux d'acides aminés acides plus faible (30,9%) dû à une diminution du taux d'acide aspartique. Les étapes du procédé selon l'invention permettent de sélectionner les peptides cationiques (riches en acides aminés basiques : arginine, histidine, lysine).

Le principe actif selon l'invention ne présente pas la caractéristique souvent revendiquée par les extraits de canneberge, à savoir le principe actif selon l'invention ne présente pas un effet anti radicalaire.

En effet, l'extrait selon l'invention ne présente pas d'activité anti radicalaire selon le test DPPH comme présenté dans le tableau 3 ci-après.

Le diphényl-picrylhydrazylhydrate (DPPH) est un radical libre, absorbant dans le violet à 517nm. Un produit anti-radicalaire entraine une disparition de la coloration violette. L'activité anti-radicalaire d'une substance est exprimée en pourcentage d'inhibition de l 'absorbance du DPPH.

**[Tableau 3]**

| | Pourcentage d'inhibition de l'absorbance du DPPH |
|---|---|
| Principe actif pur | 10% |
| 50% de principe actif | 7% |
| 10% de principe actif | 3% |

A la dose maximale recommandée c'est-à-dire 10% de principe actif, le pourcentage d'inhibition de l'absorbance du DPPH est faible. Ainsi, l'extrait selon l'invention ne présente pas d'activité anti radicalaire.

### Exemple 3 : exemple comparatif avec des extraits hors invention (HI)

Les produits suivants sont obtenus à partir des tourteaux de *Vaccinium macrocarpon* avec un procédé différent de celui de l'exemple 2.

Le produit HI 1 est obtenu avec un procédé qui diffère de celui de l'exemple 2 en ce qu'il ne contient pas d'étape d'hydrolyse.

Le produit obtenu, présente les caractéristiques analytiques suivantes :
- Une teneur en matière sèche de 26,7 g/L
- Une teneur en protéine de 1,6 g/L (6%)
- Une teneur en sucre de 11,4 g/L (57%)

Le produit présente donc une teneur en protéine de 6% par rapport à la teneur en matière sèche et donc bien inférieure au taux minimum de 80% revendiqué dans le cadre de l'invention.

Le produit HI 2 est obtenu avec un procédé qui diffère de celui de l'exemple 2 en ce qu'il ne contient pas les étapes de tri moléculaire.

Le produit obtenu, présente les caractéristiques analytiques suivantes :
- Une teneur en matière sèche de 53,8 g/L
- Une teneur en protéine de 19 g/L (35%)
- Une teneur en sucre de 17,3 g/L (32%)

Le produit présente une teneur en protéine de 35% par rapport à la teneur en matière sèche et donc inférieure au taux minimum de 80%, revendiqué dans le cadre de l'invention.

L'efficacité de l'extrait HI 2 et de celui de la présente invention ont été étudiées en suivant l'expression de gènes liés à la fonction barrière et à la dynamique matricielle, suivant le protocole décrit ci-après. Les résultats comparatifs entre l'extrait Hors-Invention et celui de la présente invention sont présentés dans le tableau 4 ci-dessous.

**[Tableau 4]**

| | | Extrait issu de l'Exemple 2 | HI 2 |
|---|---|---|---|
| Sur keratinocytes âgés | Ki67 | + | + |
| | Claudin1 | + | + |
| | IL8 | ++ | - |
| | loricrine | + | - |
| Sur fibroblastes âgés | MMP1 | + | + |
| | BGN | + | - |
| | AQP3 | + | - |

Il apparait clairement que l'extrait HI 2 issu d'un procédé différent et contenant moins de 80% de peptides, ne présente pas une efficacité semblable à celle de l'extrait selon l'invention pour l'expression des gènes étudiés.

### Exemple 4 : exemple de composition selon l'invention

Un exemple de formulation comprenant le principe actif selon l'invention sous forme de gel crème est présenté dans le tableau 5 suivant :

**[Tableau 5]**

| | **Ingrédients** | % |
|---|---|---|
| **A** | Eau | qsp 100 |
| | Glycerin | 2,00 |
| | Butylene Glycol | 3,00 |
| **B** | Carbomer | 0,30 |
| **C** | Aqua (Water) & Sodium Hydroxide | 0,20 |
| **D** | Caprylyl Methicone | 3,00 |
| | Dimethicone | 3,00 |
| | Dimethicone & Dimethicone/Vinyl Dimethicone Crosspolymer | 5,00 |
| **E** | Sodium Acryloyldimethyltaurate/VP Crosspolymer | 0,75 |
| **F** | Conservateur | qs |
| **G** | Principe actif invention | 2,00 |
| **H** | Aqua (Water) & Sodium Hydroxide | qsp pH 7,0 - 7,5 |

La composition de l'exemple 4 peut notamment être obtenue par le procédé suivant :
- Disperser B dans A sous agitation douce.
- Pré-neutraliser avec C.
- Sous forte agitation, ajouter D puis E.
- Maintenir l'agitation pendant 20min.
- Sous agitation modérée, ajouter F et G.
- Ajuster le pH avec H.

La composition est alors sous forme d'un gel crème épais et brillant, blanc cassé présentant un pH à 1 mois égal à 7,3, une viscosité (C/5rpm) égal à 44 200 cP.

### Exemple 5 : exemple de composition selon l'invention

Un exemple de formulation comprenant un principe actif selon l'invention sous forme d'une crème onctueuse est présenté dans le tableau 6 suivant :

**[Tableau 6]**

| | **Ingrédients** | **%** |
|---|---|---|
| **A1** | Eau | qsp 100 |
| | Monosodium Citrate | 0,20 |
| | Conservateur | qs |
| | Erythritol | 2,00 |
| **A2** | Butylène Glycol | 3,00 |
| | Xanthan Gum | 0,20 |
| | PEG-100 Stearate & Glyceryl Stearate | 3,00 |
| | Arachidyl Alcohol & Behenyl Alcohol & Arachidyl Glucoside | 3,00 |
| | Pentaerythrityl Distearate | 0,50 |
| | Butyrospermum Parkii (Shea) Butter | 2,00 |
| **B** | Isocetyl Stearate | 7,00 |
| | Cetearyl Ethylhexanoate | 8,00 |
| | Diisopropyl Sebacate | 4,00 |
| | Dimethicone | 3,00 |
| | Argania Spinosa Kernel Oil | 3,00 |
| | Tocopherol & Helianthus Annuus (Sunflower) Seed Oil | 0,10 |
| **C** | Hydroxyethyl Acrylate/Sodium Acryloyldimethyl Taurate Copolymer | 0,50 |
| **D** | Propylene Glycol | 2,00 |
| | Alumina & CI 77891 (Titanium Dioxide) | 1,00 |
| **E** | Principe Actif de l'invention | 2,00 |
| **F** | Aqua (Water) & Sodium Hydroxide | qs pH 5,0 - 6,0 |

La composition de l'exemple 5 peut notamment être obtenue par le procédé suivant :
- Ajouter A2 dans A1 sous agitation modérée, agiter jusqu'à obtention d'un gel homogène et chauffer à 80°C.
- Placer B sous agitation magnétique et chauffer à 80°C.
- Emulsionner B dans A avec un rotor-stator pendant 10min.
- A 50°C, sous agitation modérée ajouter C, puis D et E.
- Refroidir jusqu'à température ambiante sous agitation douce, puis ajuster le pH avec F.

La composition est alors sous forme d'une émulsion épaisse blanche et brillante, présentant un pH à 1 mois égal à 6,0, une viscosité (C/5rpm) égal à 57 000 cP.

### Exemple 6 : exemple de composition selon l'invention

Un exemple de formulation comprenant un principe actif selon l'invention sous forme d'une crème de nuit est présenté dans le tableau 7 suivant :

**[Tableau 7]**

| | **Ingrédients** | **%** |
|---|---|---|
| **A1** | Eau | qsp 100 |
| | Sodium Citrate | 0,20 |
| | Conservateur | qs |
| **A2** | Isopentyldiol | 2,00 |
| | Glycerin | 1,50 |
| | Xanthan Gum | 0,10 |
| | Cetyl Alcohol & Glyceryl Stearate & PEG-75 Stearate & Ceteth-20 & Steareth-20 | 4,00 |
| | Potassium Cetyl Phosphate | 0,75 |
| | Cetearyl Alcohol | 1,00 |
| **B** | Hydrogenated Coconut Oil | 2,00 |
| | Pentaerythrityl Tetraisostearate | 3,00 |
| | Triheptanoin | 3,00 |
| | Isopropyl Isostearate | 6,00 |
| | Isopropyl Palmitate | 6,00 |
| | Dimethicone | 3,00 |
| | Tocopherol & Helianthus Annuus (Sunflower) Seed Oil | 0,05 |
| **C** | Hydroxyethyl Acrylate/Sodium Acryloyldimethyl Taurate Copolymer & Polyisobutene & Aqua (Water) & PEG-7 Trimethylolpropane Coconut Ether & Sorbitan Isostearate | 1,00 |
| **D** | Principe Actif de l'Invention | 2,00 |

La composition de l'exemple 6 peut notamment être obtenue par le procédé suivant :
- Ajouter A2 dans A1 sous agitation modérée, agiter jusqu'à obtention d'un gel homogène et chauffer à 80°C.
- Placer B sous agitation magnétique et chauffer à 80°C.
- Emulsionner B dans A sous agitation cisaillante pendant 10min.
- A 40-50°C, sous agitation modérée ajouter C, puis D.

La composition est alors sous forme d'une émulsion épaisse blanche et brillante.

### Exemple 7 : exemple de composition selon l'invention

Un exemple de formulation comprenant un principe actif selon l'invention sous forme d'un fond de teint est présenté dans le tableau 8 suivant :

**[Tableau 8]**

| | Ingrédients | % |
|---|---|---|
| | Polyglyceryl-3 Polyricinoleate & Polyglyceryl-3 Diisostearate | 5,00 |
| | Hydrogenated Castor Oil | 0,50 |
| | Cera Alba (Beeswax) | 0,50 |
| | Dicaprylyl Carbonate & Stearalkonium Hectorite & Propylene Carbonate | 6,00 |
| **A** | Isononyl Isononanoate | 4,00 |
| | Dimethicone | 4,00 |
| | Triheptanoin | 4,00 |
| | C12-15 Alkyl Benzoate | 4,00 |
| | Magnesium Stearate | 1,00 |
| **B** | Cocoglycerides | 12,00 |
| | CI 77499 (Iron Oxides) & Hydrogenated Lecithin | 0,10 |
| | CI 77491 (Iron Oxides) & CI 77499 (Iron Oxides) & Hydrogenated Lecithin | 0,50 |
| | CI 77891 (Titanium Dioxide) & Hydrogenated Lecithin | 8,00 |
| | CI 77492 (Iron Oxides) & Hydrogenated Lecithin | 1,00 |
| **C** | Aqua (Water) | qsp 100 |
| | Glycerin | 5,00 |
| | Magnesium Sulfate | 1,00 |
| | Conservateur | qs |
| | Polyglyceryl-3 Polyricinoleate & Polyglyceryl-3 Diisostearate | |
| **D** | Principe Actif de l'Invention | 2,00 |

La composition de l'exemple 7 peut notamment être obtenue par le procédé suivant :
- Chauffer A à 85°C et homogénéiser sous agitation vive.
- Broyer B à la tricylindre et transférer dans A. Agiter jusqu'à homogénéité.
- Placer C sous agitation magnétique et chauffer à 80°C.
- Ajouter C dans AB très lentement sous agitation vive.
- Homogénéiser pendant 10min sous agitation cisaillante.
- A 30°C, ajouter D et homogénéiser pendant 2min sous agitation cissaillante.

La composition est alors sous forme d'une émulsion souple et brillante.

### Essais d'efficacité

### Essai 1 : Evaluation du potentiel d'activité du Principe actif selon l'invention sur les gènes majeurs de l'homéostasie cutanée

L'objectif de cette étude est d'évaluer le potentiel d'activité du principe actif selon l'invention sur les gènes majeurs impliqués dans l'équilibre cutané.

Cette étude a été réalisée sur 34 gènes impliqués dans les différents processus liés à l'établissement de :
- la fonction barrière épidermique : prolifération, hydratation, cohésion, différenciation, desquamation, immunité innée, système anti-oxydant et inflammation (cf. Tableau 9 : Prolifération, Hydratation, Cohésion, Différenciation, Anti radicalaire et Inflammation).
- la jonction dermo-épidermique JDE ;
- la matrice dermique : organisation et dégradation des fibres, hydratation et inflammation (cf. Tableau 9 : Organisation matricielle, dégradation matricielle, Hydratation, Protéoglycane, Facteurs de croissance et Inflammation).

L'expression des différents gènes ciblés a été évaluée par PCR quantitative sur des kératinocytes humains issus de donneurs jeunes (≤ 30 ans) et âgés (≥ 60 ans) ou des fibroblastes humains jeunes (< P5) et âgés par réplications successives (plus de 20 réplications).

Le protocole opératoire de l'étude est décrit en suivant. Les cellules humaines jeunes et âgées sont ensemencées et incubées à 37°C. Puis, les cellules sont traitées pendant 48h avec le principe actif selon l'invention ou une solution de rétinol à 0,5.10-5M.

Après plusieurs jours, les cellules sont récupérées et les ARN totaux extraits. Les ARNs ont été reverse-transcrits et les ADNs complémentaires obtenus ont été analysés par la technique de PCR quantitative. Les ARNm de témoins internes de référence, ont été analysés en parallèle des ARNm des marqueurs impliqués dans la fonction barrière de l'épiderme, dans la JDE et dans la matrice dermique.

L'incorporation de fluorescence est mesurée en continu à l'aide d'un thermocycleur. L'analyse des Ct (quantification relative) est réalisée à l'aide d'un logiciel.

Les résultats sont présentés dans le tableau 9.

**[Tableau 9]**

| | | **Cellules jeunes** | **Cellules âgés** | | | | |
|---|---|---|---|---|---|---|---|
| | Gènes | Témoin | Témoin | Principe actif de l'exemple2 0,5% | **Efficacité du PA/ Témoin âgé (%)** | Rétinol 1 µM | **Efficacité du rétinol/ Témoin âgé (%)** |
| **Prolifération** | MKI67 | 100 | 66 | 93 | +41 | 95 | +44 |
| **Hydratation** | AQP3 | 100 | 61 | 84 | +38 | 69 | +13 |
| | HAS3 | 100 | 68 | 101 | +49 | 357 | +425 |
| | CD44 | 100 | 83 | 104 | +25 | 147 | +77 |
| **Cohésion** | CLDN1 | 100 | 69 | 90 | +30 | 33 | -52 |
| | DSG | 100 | 72 | 85 | +18 | 1 | -99 |
| **Différenciation** | FLG | 100 | 38 | 56 | +47 | 9 | -76 |
| | LOR | 100 | 20 | 35 | +75 | 1 | -95 |
| | TGM1 | 100 | 68 | 89 | +31 | 47 | -31 |
| | KRT10 | 100 | 73 | 91 | +25 | 2 | -97 |
| | IVL | 100 | 53 | 64 | +21 | 120 | +126 |
| | S100A7 | 100 | 38 | 50 | +32 | 564 | +1384 |
| **Anti radicalaire** | SOD1 | 100 | 89 | 100 | +12 | 95 | +7 |
| **Inflammation** | CXCL8 | 100 | 252 | 161 | -36 | 729 | +189 |
| | S100A8 | 100 | 208 | 186 | -11 | 256 | +23 |
| | S100A9 | 100 | 191 | 177 | -7 | 397 | +108 |
| **Eléments structuraux de le JDE** | LAMA3 | 100 | 103 | 114 | +11 | 94 | -9 |
| | COL4A1 | 100 | 40 | 54 | +35 | 38 | -5 |
| | COL7A1 | 100 | 86 | 105 | +22 | 68 | -21 |
| | COL17A 1 | 100 | 97 | 113 | +16 | 81 | -16 |
| **Organisation matricielle** | COL1A1 | 100 | 63 | 78 | +24 | 79 | +25 |
| | COL3A1 | 100 | 43 | 53 | +23 | 72 | +67 |
| | POSTN | 100 | 45 | 43 | -4 | 56 | +24 |
| | ELN | 100 | 60 | 77 | +28 | 63 | +5 |
| | FBN1 | 100 | 91 | 99 | +9 | 90 | -1 |
| **Dégradation matricielle** | MMP1 | 100 | 216 | 136 | -37 | 164 | -24 |
| **Hydratation** | AQP3 | 100 | 56 | 98 | +75 | 73 | +30 |
| **Protéoglycane** | HSPG2 | 100 | 87 | 87 | 0 | 90 | +3 |
| | BGN | 100 | 63 | 77 | +22 | 78 | +24 |
| | DCN | 100 | 83 | 91 | +10 | 89 | +7 |
| | ACAN | 100 | 38 | 45 | +18 | 10 | -74 |
| | LUM | 100 | 90 | 94 | +4 | 87 | -3 |
| **Facteurs de croissance** | VEGFA | 100 | 91 | 88 | -3 | 93 | +2 |
| | TGFβ1 | 100 | 95 | 97 | +2 | 103 | +8 |
| **Inflammation** | CXCL8 | 100 | 210 | 185 | -12 | 803 | +282 |
| | IL1 | 100 | 226 | 160 | -29 | 567 | +151 |

Sur les 34 gènes évalués, 76% présentent une expression modifiée au cours du vieillissement traduisant un impact majeur sur la fonction barrière, la jonction dermo-épidermique et la matrice dermique.

Testé à 0,5% sur des kératinocytes âgés, le principe actif selon l'invention restaure significativement l'expression des gènes clés de :
- la prolifération (Ki67 : + 41%) ;
- la différenciation (loricrine : + 75% et filaggrine : +47%);
- la cohésion (claudine-1 : +30%) ;
- la défense anti-radicalaire (SOD1 : +12%).

Testé à 0,5% sur des fibroblastes âgés, le principe selon l'invention agit significativement sur l'expression des gènes liés à l'organisation matricielle (collagène 1 : +24% et élastine : +28%) et à sa dégradation (MMP1 : -37%). Sur les deux types cellulaires, une action significative a été mise en évidence sur les marqueurs liés à l'hydratation et l'inflammation. Le principe actif selon l'invention et le rétinol présentent des effets similaires sur les gènes liés à la matrice.

### Essai 2 : Efficacité biologique du principe actif selon l'invention sur la fonction barrière de l'épiderme et l'hydratation.

L'objectif de cette étude est d'évaluer la capacité du principe actif selon l'invention à maintenir une fonction barrière fonctionnelle en stimulant la synthèse de Ki 67, de loricrine, de claudine-1 et d'aquaporine 3.

Une des stratégies majeures de la peau pour se protéger est de maintenir une barrière intacte. Cette barrière, issue du processus de prolifération et différenciation des kératinocytes, résulte de la structure particulière du *stratum corneum* et de la cohésion des couches supérieures de l'épiderme assurée par les jonctions serrées. Plusieurs composants protéiques sont indispensables à l'établissement de cette fonction barrière tels que :
- Le Ki-67 qui est un marqueur clé de la prolifération des cellules de la couche basale de l'épiderme.
- La loricrine qui joue un rôle majeur dans la différenciation terminale.
- La claudine-1 qui est un des constituants principaux des jonctions serrées, structures complexes qui établissent une véritable cohésion cellule-cellule.
- L'aquaporine 3 qui fait partie des protéines membranaires essentielles au transport de l'eau et permet de maintenir l'hydratation de la couche cornée.

La prolifération, la cohésion et le niveau d'hydratation sont ainsi directement liés au processus de différenciation épidermique et à l'état de la fonction barrière.

Cette étude a été réalisée sur des épidermes reconstruits normaux et âgés. La construction épidermique a été évaluée à l'aide d'une coloration Hématoxyline Eosine (HE) et la synthèse des marqueurs de la fonction barrière par immunohistofluorescence.

Le protocole opératoire de l'étude est décrit en suivant. Les kératinocytes humains jeunes et âgés sont ensemencés sur des inserts puis incubés à 37°C dans une atmosphère contenant 5% de CO2 et cultivés pendant plusieurs jours.

Les épidermes reconstruits sont traités pendant 48 heures en systémique avec :
- Le principe actif selon l'invention à 0,1% et 0,5% (V/V)
- Une solution de rétinol à 10 µM.

Les épidermes reconstruits sont récupérés, fixés, déshydratés et inclus en paraffine. Des coupes (4 µm) sont ensuite réalisées à l'aide d'un microtome.

L'analyse de l'épaisseur des épidermes reconstruits est réalisée par coloration hématoxyline éosine (HE).

L'analyse de la synthèse de Ki67, claudine-1, aquaporine 3 et loricrine est réalisée par immunohistofluorescence.

La visualisation est réalisée à l'aide d'un microscope couplé à un système d'analyse d'images. L'épaisseur des épidermes obtenus a été mesurée sur les coupes histologiques réalisées.

Le taux des différents marqueurs synthétisés est proportionnel à l'intensité de fluorescence verte présente sur les épidermes reconstruits. Une analyse quantitative des images a été réalisée grâce à un script d'analyse d'images. Les résultats sont exprimés en unités arbitraires (UA).

Les résultats concernant la capacité du principe actif selon l'invention et du rétinol à restaurer l'épaisseur de l'épiderme sont présentés dans le tableau 10.

**[Tableau 10]**

| | **Mesure de l'épaisseur de l'épiderme (µm)** | **Capacité à restaurer l'épaisseur de l'épiderme (%)** |
|---|---|---|
| **ép idermes reconstruits jeunes** | | |
| Témoin | 60 | |

| **épidermes reconstruits âgés** | | |
|---|---|---|
| Témoin | 44 | |
| Principe Actif 0,1% | 54 | +63 |
| Principe Actif 0,5% | 60 | +100 |
| Rétinol 10 µM | 59 | +94 |

Les résultats concernant la capacité du principe actif selon l'invention à restaurer la synthèse de Ki-67 sont présentés dans le tableau 11.

**[Tableau 11]**

| | **Cellules Ki-67 positives (%)** | **Capacité à restaurer la synthèse de Ki-67 (%)** |
|---|---|---|
| **épidermes reconstruits jeunes** | | |
| Témoin | 27 | |

| **épidermes reconstruits âgés** | | |
|---|---|---|
| Témoin | 13 | |
| Principe Actif 0,1% | 21 | +57 |
| Principe Actif 0,5% | 25 | +86 |
| Rétinol 10 µM | 23 | +71 |

Les résultats concernant la capacité du principe actif selon l'invention à restaurer la synthèse de loricrine sont présentés dans le tableau 12.

**[Tableau 12]**

| | **Synthèse de loricrine (x10⁴ UA)** | **Capacité à restaurer la synthèse de loricrine (%)** |
|---|---|---|
| **épidermes reconstruits jeunes** | | |
| Témoin | 372 | |

| **épidermes reconstruits âgés** | | |
|---|---|---|
| Témoin | 191 | |
| Principe Actif 0,1% | 256 | +36 |
| Principe Actif 0,5% | 279 | +49 |
| Rétinol 10 µM | 177 | 0 |

Les résultats concernant la capacité du principe actif selon l'invention à restaurer la synthèse de claudine-1 sont présentés dans le tableau 13.

**[Tableau 13]**

| | **Synthèse de claudine-1 (x10⁴ UA)** | **Capacité à restaurer la synthèse de claudine-1 (%)** |
|---|---|---|
| **épidermes reconstruits jeunes** | | |
| Témoin | 270 | |

| **épidermes reconstruits âgés** | | |
|---|---|---|
| Témoin | 180 | |
| Principe Actif 0,1% | 223 | +48 |
| Principe Actif 0,5% | 243 | +70 |
| Rétinol 10 µM | 136 | 0 |

Les résultats concernant la capacité du principe actif selon l'invention à restaurer la synthèse d'aquaporine 3 sont présentés dans le tableau 14.

**[Tableau 14]**

| | **Synthèse d'aquaporine** 3 **(x10⁴ UA)** | **Capacité à restaurer la synthèse d'aquaporine 3 (%)** |
|---|---|---|
| **épidermes reconstruits jeunes** | | |
| Témoin | 355 | |

| **épidermes reconstru**its **âgés** | | |
|---|---|---|
| Témoin | 249*^{✧✧}* | |
| Principe Actif 0,1% | 308* | +56 |
| Principe Actif 0,5% | 327* | +74 |
| Rétinol 10 µM | 355** | +100 |

Les épidermes reconstruits âgés présentent une fonction barrière altérée, caractérisée par une diminution significative de l'épaisseur de l'épiderme, de la synthèse de Ki-67, de loricrine, de claudine-1, et d'aquaporine 3.

Testé à 0,50% sur des épidermes reconstruits âgés, le principe actif selon l'invention restaure significativement l'épaisseur de l'épiderme et la synthèse de :
- Ki-67 de +86% ;
- loricrine de +49% ;
- claudine-1 de +70% ;
- aquaporine 3 de +74%.

Le principe actif selon l'invention permet ainsi de maintenir une barrière fonctionnelle et hydratée.

### Essai 3 : Efficacité biologique du principe actif selon l'invention sur la dynamique matricielle.

L'objectif de cette étude est d'évaluer la capacité du principe actif selon l'invention à favoriser la production d'un réseau matriciel fonctionnel.

La matrice extracellulaire dermique est constituée principalement d'un réseau dense de fibres de collagène I. Ce collagène joue un rôle clef dans toutes les phases de remodelage tissulaire. A partir de 50 ans, la qualité du derme se détériore progressivement. Le réseau de fibres de collagène est de moins en moins dense et de plus en plus fragmenté. Cet effet est notamment imputable à l'augmentation de la métalloprotéinase matricielle MMP-1 qui dégrade les fibres de collagène I et III. Par ailleurs, l'acide hyaluronique est essentiel au maintien de la structure des couches de la peau. Il se combine avec l'eau et remplit les espaces intercellulaires, ce qui contribue à son pouvoir hautement hydratant. La quantité et la qualité d'acide hyaluronique, principal glycosaminoglycane dans le derme, diminuent progressivement avec l'âge.

L'effet du principe actif selon l'invention sur :
- le réseau de collagène I a été étudié par immunocytologie, sur des fibroblastes humains jeunes (P5) et âgés (P22) obtenus par réplications successives ;
- le taux de MMP 1 a été mesuré par dosage ELISA sur des fibroblastes humains issus de donneurs âgés de plus de 60 ans et soumis une exposition aux UVA ;
- la synthèse d'acide hyaluronique a été évaluée par dosage ELISA sur des fibroblastes humains issus de donneurs âgés de plus de 60 ans

### Etude du réseau de collagène I

Les fibroblastes humains sont ensemencés et incubés à 37°C dans une atmosphère contenant 5% de CO2.

Les cellules sont traitées tous les deux jours avec :
- Le principe actif selon l'invention à 0,05% et 0,10% (V/V)
- une solution de rétinol à 0,1µM

Elles sont ensuite incubées dans une atmosphère contenant 5% de CO2 à 37°C.

Le marquage immunocytologique du collagène I est réalisé par fixation et perméabilisation à l'aide d'anticorps primaire I et d'anticorps secondaire couplé à un fluorophore.

Une analyse quantitative a été réalisée par lecture de la fluorescence des différentes conditions testées à l'aide d'un fluorimètre. L'intensité du réseau de collagène I est proportionnelle à l'intensité de fluorescence verte présente. Les résultats sont exprimés en valeur de fluorescence (UA).

### Etude du taux de MMP-1

Les fibroblastes humains âgés sont ensemencés et incubés à 37°C dans une atmosphère contenant 5% de CO2. Les irradiations sont réalisées à l'aide d'une lampe UV avec des intensités UVA de 10 J/cm2.

Le milieu de culture est remplacé par du milieu contenant :
- Le principe actif selon l'invention à 0,5% et 1% (V/V)
- Une solution de rétinol à 1µM

Les cellules sont ensuite incubées à 37°C dans une atmosphère contenant 5% de CO2 pendant 48 heures.

### Etude du taux d'acide hyaluronique

Les fibroblastes humains âgés sont ensemencés et incubés à 37°C dans une atmosphère contenant 5% de CO2.

Le milieu de culture est remplacé par du milieu contenant :
- Le principe actif selon l'invention à 0,5% et 1% (V/V)
- une solution de rétinol à 1µM

Les cellules sont ensuite incubées à 37°C dans une atmosphère contenant 5% de CO2 pendant 48 heures.

Les résultats concernant la capacité du principe actif selon l'invention et du rétinol à restaurer la synthèse du réseau de collagène I dans des fibroblastes âgés sont présentés dans le tableau 15.

**[Tableau 15]**

| | **Synthèse du réseau de collagène I (x10³ UA)** | **Capacité à restaurer la synthèse du réseau de collagène I (%)** |
|---|---|---|
| **Fibroblastes jeunes** | | |
| Témoin | 240 | |

| **Fibroblastes âgés** | | |
|---|---|---|
| Témoin | 177 | |
| Principe Actif 0,05% | 194 | +27 |
| Principe Actif 0,10% | 211 | +54 |
| Rétinol 0,1 µM | 174 | |

Les résultats concernant l'effet du Principe actif selon l'invention sur la synthèse des MMP-1 par les fibroblastes humains âgés soumis à une irradiation UVA sont présentés dans le tableau 16.

**[Tableau 16]**

| | **Taux de MMP-1 (ng/mg protéines)** | **Capacité à réduire le taux de MMP-1 (%)** |
|---|---|---|
| **Fibroblastes non irradiés** | | |
| Témoin | 12 | |

| **Fibrobl astes irradiés** | | |
|---|---|---|
| Témoin | 63 | |
| Principe Actif 0,5% | 41 | +43 |
| Principe Actif 1% | 40 | +45 |
| Rétinol 1 µM | 46 | +33 |

Les résultats concernant l'effet du principe actif selon l'invention sur la synthèse de l'acide hyaluronique par des fibroblastes humains âgés soumis à une irradiation UVA sont présentés dans le tableau 17.

**[Tableau 17]**

| | **Synthèse d'acide hyaluronique (ng/mg protéines)** | **Taux d'acide hyaluronique / Témoin (%)** |
|---|---|---|
| **Fibroblastes âgés** | | |
| Témoin | 3804 | |
| Principe Actif 0,5% | 4678 | + 23 |
| Principe Actif 1% | 5066 | + 33 |
| Rétinol 1 µM | 4112 | +8 |

Les fibroblastes âgés présentent un réseau matriciel altéré, caractérisé par une diminution significative du réseau de collagène I et de la synthèse d'acide hyaluronique. En réponse à des irradiations UVA, ils produisent de manière significative des MMP-1.

Testé à 0,1%, le principe actif selon l'invention :
- augmente significativement la production du réseau de collagène I de 54%.

Testé à 1%, le principe actif selon l'invention :
- diminue significativement la synthèse des MMP-1 par des fibroblastes âges irradiés de 45%.
- augmente significativement la synthèse d'acide hyaluronique de 33%.

Le principe actif selon l'invention maintient ainsi une organisation fonctionnelle des fibres de la matrice et contribue à l'hydratation dans le derme.

### Essai 4 : Efficacité cosmétique du principe actif selon l'invention évaluée chez des volontaires sains caucasiens et asiatiques (Test in vivo).

L'objectif de cette étude est d'évaluer in vivo les bénéfices cosmétiques du principe actif selon l'invention formulé à 2% en émulsion, en comparaison à une formule placebo, chez des volontaires caucasiens et asiatiques.

Le panel caucasien est composé de 2 groupes de volontaires sains, de sexe féminin, âgés de 45 à 66 ans présentant des rides au niveau de la patte d'oie et ayant appliqué le placebo et le principe actif selon l'invention 2% en hémi-visage de façon randomisée et biquotidienne pendant 42 jours.

Les bénéfices cosmétiques du principe actif selon l'invention ont été évalués sur panel caucasien au niveau du visage, après 21 et 42 jours d'application biquotidienne, selon les méthodes suivantes :
- Etude de l'éclat du teint par scorage visuel par experts (rayonnement, couleur rose, couleur olive, état de fatigue des yeux)
- Etude de l'effet anti-rides par projection de franges.

Le panel asiatique est composé de 2 groupes de volontaires sains, de sexe féminin, présentant des rides au niveau de la patte d'oie et un teint terne, ayant appliqué soit le principe actif de l'invention à 2%, soit le placebo, en visage entier, de façon biquotidienne pendant 42 jours.

Les bénéfices cosmétiques du principe actif de l'invention ont été évalués sur panel asiatique au niveau du visage, après 21 et 42 jours d'application biquotidienne, selon les méthodes suivantes :
- Etude de l'éclat du teint par scorage clinique.
- Etude de l'effet anti-rides par scorage clinique.

### A) Effet du principe actif selon l'invention sur l'éclat du teint

Le principe actif selon l'invention formulé à 2% en émulsion, améliore les paramètres caractéristiques de l'éclat du teint chez des sujets caucasiens. Cet effet apparaît de façon importante et significative dès 21 jours.

En effet, comparé au placebo, le principe actif selon l'invention :
- donne un teint plus lumineux et plus frais en augmentant significativement le rayonnement de la peau de 12%. Cet effet a été observé chez 89% des volontaires et augmente après 42 jours de traitement (+14%) chez 88% des volontaires.
- augmente également la couleur rose de manière significative de 17%. Cet effet a été observé chez 61% des volontaires et augmente après 42 jours de traitement (+22%) chez 82% des volontaires.
- diminue significativement la couleur olive de 8%. Cet effet a été observé chez 61% des volontaires et s'intensifie après 42 jours de traitement (-12%) chez 88% des volontaires.
- diminue également l'état de fatigue des yeux de 8%. Cet effet a été observé chez 72% des volontaires et s'intensifie après 42 jours de traitement (-10%) chez 82% des sujets.

Le principe actif selon l'invention formulé à 2% en émulsion, améliore également les paramètres caractéristiques de l'éclat du teint chez des sujets asiatiques. Cet effet apparaît de façon significative dès 21 jours et s'intensifie après 42 jours de traitement.

En effet, comparé au placebo, le principe actif selon l'invention :
- augmente significativement le rayonnement de la peau de 5%, cet effet s'améliore encore après 42 jours de traitement (+10%)
- augmente la luminosité de 6%, cet effet s'améliore encore après 42 jours de traitement (+8%),
- augmente la couleur rose, relié à la fraicheur du teint de 9%, cet effet augmente après 42 jours de traitement de 18%.
- entraîne une diminution de la couleur olive (-5%), ce paramètre diminue encore après 42 jours de traitement de 12%.

Ces effets ont été observés chez 97% des volontaires.

### B) Effet anti-rides du principe actif selon l'invention

Le principe actif selon l'invention formulé à 2% présente un effet anti-rides rapide et significatif chez des sujets caucasiens dès 21 jours d'utilisation biquotidienne.

En effet, en comparaison au placebo, le principe actif selon l'invention :
- lisse le relief cutané des pattes d'oie en réduisant significativement, dès 21 jours, la rugosité de la peau (paramètre Sa =-6%). Cet effet se maintient après 42 jours d'utilisation (Sa = -7%).
- réduit les rides en permettant une diminution du paramètre volume négatif (-22% après 21 jours de traitement et -19% après 42 jours).

L'étude de la répartition des résultats montre une amélioration de ces paramètres chez 83% des sujets.

De même, le principe actif selon l'invention formulé à 2% présente un effet anti-rides rapide et significatif chez des sujets asiatiques dès 21 jours d'utilisation biquotidienne.

En effet, en comparaison au placebo, le principe actif selon l'invention diminue le stade de rides de la patte d'oie de 10% et ce, dès 21 jours de traitement. Cet effet s'intensifie après 42 jours d'utilisation (diminution du stade de rides de 20%). Cet effet a été observé chez respectivement 53% et 84% des volontaires.

### Essai 5 : Efficacité du principe actif selon l'invention sur la barrière cutanée chez des volontaires sains caucasiens et asiatiques.

L'objectif de cette étude est d'évaluer in vivo l'effet du principe actif selon l'invention, formulé à 2,5% en émulsion, sur la qualité de la barrière cutanée, en comparaison à une formule placebo, chez des volontaires caucasiens et asiatiques.

Le panel caucasien est composé de deux groupes de 18 volontaires sains, de sexe féminin, âgés de 45 à 66 ans, présentant des rides au niveau de la patte d'oie et ayant appliqué matin et soir le principe actif selon l'invention et le placebo en hémi-visage de façon randomisée et biquotidienne pendant 42 jours.

Le panel asiatique est composé de deux groupes de 32 volontaires sains, de sexe féminin, d'âge compris entre 41 et 64 ans, ayant appliqué matin et soir soit le principe actif selon l'invention soit le placebo en visage entier pendant 42 jours.

Des mesures de perte insensible en eau (PIE) au niveau des joues ont été réalisées à l'aide d'un Tewamètre^{®} avant et après 21 et 42 jours d'application biquotidien.

Dès 21 jours d'application biquotidienne et en comparaison au groupe placebo, le principe actif selon l'invention formulé à 2% en émulsion améliore significativement la qualité de la barrière cutanée de volontaires caucasiens (réduction des pertes en eau de 17%). Cette diminution a été observée chez 94% des volontaires.

Cet effet se maintient après 42 jours de traitement avec une diminution de la PIE égale à 15% et est observé chez 82% des sujets.

Dès 21 jours d'application biquotidienne en visage entier et en comparaison au groupe placebo, le principe actif selon l'invention formulé à 2% en émulsion améliore significativement la qualité de la barrière cutanée de volontaires asiatiques en diminuant les pertes insensibles en eau de 13%. Cette diminution a été observée chez 55% des volontaires.

Cet effet s'intensifie après 42 jours de traitement avec une diminution de la PIE égale à 15% et est observé chez 71% des sujets.

En réduisant les pertes en eau, le principe actif selon l'invention testé à 2% favorise le renforcement de la barrière cutanée des volontaires caucasiens et asiatiques.

Essai 6 : Efficacité du principe actif selon l'invention sur la qualité de la jonction dermo-épidermique chez des volontaires sains caucasiens.

L'objectif de cette étude est d'évaluer *in vivo* l'effet du principe actif selon l'invention formulé à 2% en émulsion, sur la qualité de la zone de la JDE, zone essentielle aux échanges entre le derme et l'épiderme, en comparaison à une formule placebo, chez des volontaires caucasiens.

Le panel caucasien est composé de 2 groupes de 18 volontaires sains, de sexe féminin, âgés de 45 à 66 ans présentant des rides au niveau de la patte d'oie et ayant appliqué le placebo et le principe actif selon l'invention 2% en hémi-visage de façon randomisée et biquotidienne pendant 42 jours.

L'effet du principe actif selon l'invention sur la qualité de la zone de la Jonction Dermo-Epidermique (JDE) a été évalué au niveau des joues, en comparaison au placebo, après 21 et 42 jours d'application biquotidienne, par l'étude de la structure de la jonction dermo-épidermique (LC-OCT).

En comparaison au placebo, le principe actif selon l'invention formulé à 2% en émulsion favorise l'aspect sinusoïdal de la JDE. Cet effet apparaît dès 21 jours d'application biquotidienne (+3%, effet observé sur 78% des volontaires) et s'intensifie de manière significative après 42 jours de traitement (+4%, effet observé sur 88% des volontaires).

En améliorant la sinuosité de la JDE, le principe actif selon l'invention augmente la surface de la JDE et favorise ainsi les échanges entre l'épiderme et le derme et son implication dans la résistance aux contraintes mécaniques.

Efficacité du principe actif selon l'invention sur la qualité de la matrice dermique chez des volontaires sains caucasiens.

L'objectif de cette étude est d'évaluer *in vivo* l'effet du principe actif selon l'invention formulé à 2% en émulsion sur la qualité de la matrice dermique, en comparaison à une formule placebo, chez des volontaires caucasiens.

Le panel caucasien est composé de 2 groupes de 18 volontaires sains, de sexe féminin, âgés de 45 à 66 ans présentant des rides de la patte d'oie et ayant appliqué le placebo et le principe actif selon l'invention 2% en hémi-visage de façon randomisée et biquotidienne pendant 42 jours.

L'effet du principe actif selon l'invention sur la qualité de la matrice dermique a été évalué au niveau des joues, en comparaison au placebo, après 21 et 42 jours d'application biquotidienne, par l'étude de la qualité des fibres (LC-OCT).

Dès 21 jours d'application biquotidienne et en comparaison au groupe placebo, le principe actif selon l'invention formulé à 2% en émulsion, améliore la qualité des fibres pour l'ensemble des volontaires caucasiens de 15%. Cet effet se maintient après 42 jours de traitement (+11%) et a été observé chez 94% des volontaires.

En renforçant le tissu de soutien, le principe actif selon l'invention limite ainsi le relâchement de la peau.

Essai 7 : Essai comparatif entre le principe actif selon l'invention et une molécule de référence : le rétinol.

L'objectif de cette étude est d'évaluer in vivo les bénéfices cosmétiques du principe actif selon l'invention formulé à 2% en émulsion chez des volontaires caucasiens en comparaison à une molécule de référence, le rétinol formulé à 0,075%.

Cette étude a été menée sur 3 groupes de 18 volontaires sains, de sexe féminin, âgés de 45 à 66 ans présentant des rides au niveau de la patte d'oie. Chacun des volontaires a appliqué deux des trois formules en hémi-visage de façon randomisée et biquotidienne pendant 42 jours.

L'étude des bénéfices cosmétiques a été réalisée après 21 et 42 jours de traitement selon les méthodes suivantes :
- Etude de l'éclat du teint par scorage visuel par experts.
- Etude de l'action anti-rides par projection de franges.

### Etude de l'éclat du teint

L'éclat du teint est évalué par des experts selon les paramètres suivants, rayonnement, couleur rose, couleur olive et l'état de fatigue des yeux.

Les résultats sont présentés dans le tableau 17.

**[Tableau 18]**

| | **J21** | | **J42** | |
|---|---|---|---|---|
| | **Rétinol 0,075%** | **Principe actif Invention à 2%** | **Rétinol 0,075%** | **Principe actif Invention à 2%** |
| **Rayonnement** | +6% | +12% | +11% | +14% |
| **Couleur rose** | +14% | +17% | +19% | +22% |
| **Couleur olive** | -11% | -8% | -10% | -12% |
| **Fatigue des yeux** | -2% | -8% | -10% | -10% |

Le principe actif selon l'invention présente un effet plus rapide que le rétinol sur les paramètres rayonnement, couleur rose et fatigue des yeux que le rétinol seul dès J21. Cet effet s'intensifie après 42 jours d'utilisation.

### Etude de l'effet anti-rides

L'effet anti-rides est évalué selon le paramètre de rugosité Sa, évalué sur la zone de la patte d'oie par projection de franges et sur le volume négatif, évalué sur la même zone.

Les résultats sont présentés dans le tableau 19.

**[Tableau 19]**

| | **J21** | | **J42** | |
|---|---|---|---|---|
| | **Rétinol 0,075%** | **Principe actif Invention à 2%** | **Rétinol 0,075%** | **Principe actif Invention à 2%** |
| **Paramètre Sa** | -5% | -6% | -7% | -7% |
| **Volume négatif** | -12% | -22% | -20% | -19% |

En comparaison au placebo, le rétinol formulé à 0,075% tend à diminuer les rides de la patte d'oie après 21 jours de traitement (différence non significative). Ce n'est qu'après 42 jours de traitement que le rétinol commence à présenter un effet anti-rides significatif (-20%).

Dans les mêmes conditions, le principe actif selon l'invention présente un effet anti-rides significatif plus important dès 21 jours (respectivement -22%). Cet effet se maintient après 42 jours d'application, (-19%).

Le principe actif selon l'invention présente un effet anti-rides rapide, il permet d'obtenir un effet comparable au rétinol seul.

Essai 8 : Etude de la qualité de la jonction dermo-épidermique.

L'objectif de cette étude est d'évaluer in vivo l'effet du principe actif selon l'invention formulé à 2% en émulsion sur la qualité de la zone de la JDE, zone essentielle aux échanges entre le derme et l'épiderme chez des volontaires caucasiens en comparaison à une molécule de référence, le rétinol formulé à 0,075%.

Cette étude a été menée sur 3 groupes de 18 volontaires sains, de sexe féminin, âgés de 45 à 66 ans présentant des rides au niveau de la patte d'oie. Chacun des volontaires a appliqué deux des trois formules à l'étude en hémi-visage de façon randomisée et biquotidienne pendant 42 jours.

Des acquisitions réalisées par LC-OCT après 21 et 42 jours de traitement au niveau des joues ont permis d'évaluer la structure de la jonction dermo-épidermique.

Un résumé des résultats correspondant à l'effet des différentes formules à l'étude sur la sinuosité de la JDE, étudiée par LC-OCT, est présenté dans le tableau 20.

**[Tableau 20]**

| | **J21** | | **J42** | |
|---|---|---|---|---|
| | **Rétinol 0,075%** | **Principe actif Invention à 2%** | **Rétinol 0,075%** | **Principe actif Invention à 2%** |
| **Sinuosité** | +2% | +3% | +3% | +4% |

Le principe actif selon l'invention formulé à 2% ou le rétinol à 0,075% présentent des résultats similaires en comparaison au placebo.

Dès 21 jours d'application la sinuosité de la JDE est intensifiée (principe actif selon l'invention : +3% ; rétinol : +2%). Cet effet devient significatif après de 42 jours d'application (principe actif selon l'invention : +4% ; rétinol : +3%).

Essai 9 : Etude de la qualité de la matrice dermique

L'objectif de cette étude est d'évaluer in vivo l'effet du principe actif selon l'invention formulé à 2% en émulsion sur la qualité de la matrice dermique chez des volontaires caucasiens en comparaison à une molécule de référence, le rétinol formulé à 0,075%.

Cette étude a été menée sur 3 groupes de 18 volontaires sains, de sexe féminin, âgés de 45 à 66 ans présentant des rides au niveau de la patte d'oie. Chacun des volontaires a appliqué deux des trois formules à l'étude en hémi-visage de façon randomisée et biquotidienne pendant 42 jours.

Des acquisitions réalisées par LC-OCT après 21 et 42 jours de traitement au niveau des joues ont permis d'évaluer la structure de la qualité des fibres.

Les résultats correspondant à l'effet des différentes formules à l'étude sur la qualité des fibres, étudiée par LC-OCT sont présentés dans le tableau 21.

**[Tableau 21]**

| | **J21** | | **J42** | |
|---|---|---|---|---|
| | **Rétinol 0,075%** | **Principe actif Invention à 2%** | **Rétinol 0,075%** | **Principe actif Invention à 2%** |
| Qualité des fibres | +3% | +15% | +12% | +11% |

Dès 21 jours, en comparaison au placebo, le principe actif selon l'invention à 2% améliore significativement la qualité des fibres (+15%). A ce temps, cette amélioration n'est pas visible chez le groupe ayant appliqué le rétinol à 0,075% (+3%, non significatif).

Après 42 jours de traitement, le principe actif selon l'invention et le rétinol présentent des améliorations comparables.

Ainsi, le principe actif selon l'invention améliore la qualité des fibres et ce de façon significativement plus rapide que le rétinol (différence significative à 21 jours, p=0,0020).

Essai 10 : Etude des effets indésirables.

L'utilisation du rétinol s'accompagne d'effets indésirables tels qu'une altération de la qualité de la fonction barrière et des phénomènes d'irritations cutanées.

L'objectif de cette étude est d'évaluer *in vivo* la capacité du principe actif selon l'invention formulé à 2% en émulsion et du rétinol formulé à 0,075% sur les effets indésirables du rétinol chez des volontaires caucasiens.

Cette étude a été menée sur 3 groupes de 18 volontaires sains, de sexe féminin, âgés de 45 à 66 ans présentant des rides de la patte d'oie. Chacun des volontaires a appliqué deux des trois formules, en hémi-visage, de façon randomisée et biquotidienne pendant 42 jours.

Les effets du principe actif selon l'invention et du rétinol ont été observés selon les méthodes suivante :
- Etude de la fonction barrière (Téwamètre^{®})
- Etude des irritations cutanées (C-Cube)
- Enregistrement des réactions cutanées survenues au cours de l'étude

Les résultats correspondant à l'effet des différentes formules sur la fonction barrière et sur les irritations cutanées sont présentés dans le tableau 22.

**[Tableau 22]**

| | **J21** | | **J42** | |
|---|---|---|---|---|
| | **Rétinol 0,075%** | **Principe actif Invention à 2%** | **Rétinol 0,075%** | **Principe actif Invention à 2%** |
| Perte en eau | +24% | -17% | +15% | -15% |
| Paramètre a* | +5% | +1% | +5% | 0% |

En comparaison au placebo, la formule contenant le rétinol formulé à 0,075% entraîne une diminution des pertes en eau préservant ainsi la qualité de la barrière cutanée. A l'inverse, le rétinol formulé à 0,075% provoque une augmentation des pertes en eau (+24% à J21 et +15% à J42).

Dès 21 jours de traitement, l'application de la formule contenant 0,075% de rétinol augmente de façon significative le paramètre a*, traduisant ainsi l'apparition de rougeurs cutanées. Comparé au rétinol, le principe actif selon l'invention à 2% n'occasionne pas d'apparition de rougeurs après 42 jours d'utilisation.

Enfin, dans le groupe placebo (18 volontaires inclus) et dans le groupe du principe actif selon l'invention (18 volontaires inclus) aucun sujet n'a manifesté d'effet indésirable. Dans le groupe rétinol (18 volontaires inclus), trois sujets ont manifesté des effets indésirables avec une imputabilité « vraisemblable » au produit à l'étude. Le taux d'incident dans ce groupe représente 17%.

Dans les conditions de cette étude, dès 21 jours de traitement, la formule contenant du rétinol a entrainé des effets indésirables.

Aucun effet indésirable n'a été recensé avec la formule contenant le principe actif selon l'invention à 2%.

## Revendications

1. Principe actif cosmétique comprenant au moins un extrait de *Vaccinium macrocarpon* **caractérisé en ce que** ledit extrait comprend au moins 80% de peptides en poids de matières sèches de l'extrait.

2. Principe actif cosmétique selon la revendication précédente, **caractérisé en ce que** ledit extrait est obtenu à partir du fruit de *Vaccinium macrocarapon.*

3. Principe actif cosmétique selon l'une des revendications précédentes, **caractérisé en ce que** ledit extrait est obtenu à partir des tourteaux de *Vaccinium Macrocarpon.*

4. Principe actif cosmétique selon l'une des revendications précédentes, **caractérisé en ce que** l'extrait est un hydrolysat de *Vaccinium Macrocarpon.*

5. Principe actif cosmétique selon la revendication précédente, **caractérisé en ce que** l'hydrolysat est un hydrolysat enzymatique.

6. Principe actif cosmétique selon l'une des revendications 4 ou 5, **caractérisé en ce que** l'hydrolysat comprend au moins 80% de peptides, en poids de matière sèches de l'extrait, lesdits peptides ayant une taille inférieure ou égale à 2 000 Da.

7. Principe actif cosmétique selon la revendication précédente, **caractérisé en ce que** la taille est comprise entre 243 et 2 000 Da.

8. Principe actif cosmétique selon l'une des revendications précédentes, **caractérisé en ce que** les peptides présents dans l'hydrolysat comprennent en moyenne entre 4 et 6 acides aminés.

9. Principe actif cosmétique selon la revendication précédente, **caractérisé en ce que** ledit extrait présente un taux de peptides cationiques riches en acides aminés basique supérieur à 20%.

10. Principe actif cosmétique selon l'une des précédentes revendications, **caractérisé en ce que** ledit extrait est susceptible d'être obtenu par un procédé comprenant les étapes suivantes :
- solubilisation d'au moins 100 g/L de tourteaux de *Vaccinium Macrocarpon* dans l'eau,
- hydrolyse enzymatique,
- purification de la fraction peptidique.

11. Principe actif cosmétique selon l'une des revendications précédentes, **caractérisé en ce que** ledit extrait est susceptible d'être obtenu par un procédé d'extraction comprenant deux hydrolyses enzymatiques de tourteaux de *Vaccinium Macrocarpon.*

12. Composition cosmétique pour une application topique comprenant au moins un principe actif selon l'une des revendications précédentes et un milieu physiologiquement acceptable.

13. Composition cosmétique selon la revendication précédente, dans laquelle le principe actif représente au moins 0,1% en poids du poids total de la composition.

14. Utilisation cosmétique d'un principe actif selon l'une des revendications 1 à 11 ou d'une composition cosmétique selon l'une des revendications 12 ou 13, pour un effet anti-âge.

15. Utilisation cosmétique selon la revendication précédente pour un effet anti-rides et/ou améliorer l'éclat du teint et/ou améliorer la qualité de la jonction dermo-épidermique et/ou améliorer l'homéostasie cutanée.

## Patentansprüche

1. Kosmetischer Wirkstoff, umfassend mindestens einen Extrakt von *Vaccinium macrocarpon,* **dadurch gekennzeichnet, dass** der Extrakt mindestens zu 80 % Peptide umfasst, bezogen auf das Gewicht der Trockenmasse des Extrakts.

2. Kosmetischer Wirkstoff nach dem vorstehenden Anspruch, **dadurch gekennzeichnet, dass** der Extrakt aus der Frucht von *Vaccinium macrocarpon* gewonnen wird.

3. Kosmetischer Wirkstoff nach einem der vorstehenden Ansprüche, **dadurch gekennzeichnet, dass** der Extrakt aus Presskuchen von *Vaccinium macrocarpon* gewonnen wird.

4. Kosmetischer Wirkstoff nach einem der vorstehenden Ansprüche, **dadurch gekennzeichnet, dass** der Extrakt ein Hydrolysat von *Vaccinium macrocarpon* ist.

5. Kosmetischer Wirkstoff nach dem vorstehenden Anspruch,
**dadurch gekennzeichnet, dass** das Hydrolysat ein enzymatisches Hydrolysat ist.

6. Kosmetischer Wirkstoff nach einem der Ansprüche 4 oder 5, **dadurch gekennzeichnet, dass** das Hydrolysat mindestens zu 80 % Peptide umfasst, bezogen auf das Gewicht der Trockenmasse des Extrakts, wobei die Peptide eine Größe von mehr als oder gleich 2.000 Da vorweisen.

7. Kosmetischer Wirkstoff nach dem vorstehenden Anspruch, **dadurch gekennzeichnet, dass** die Größe zwischen 243 und 2000 Da liegt.

8. Kosmetischer Wirkstoff nach einem der vorstehenden Ansprüche, **dadurch gekennzeichnet, dass** die Peptide, die in dem Hydrolysat enthalten sind, im Durchschnitt zwischen 4 und 6 Aminosäuren umfassen.

9. Kosmetischer Wirkstoff nach dem vorstehenden Anspruch, **dadurch gekennzeichnet, dass** der Extrakt einen Anteil an kationischen Peptiden, die reich an basischen Aminosäuren sind, von über 20 % aufweist.

10. Kosmetischer Wirkstoff nach einem der vorstehenden Ansprüche, **dadurch gekennzeichnet, dass** der Extrakt geeignet ist, um durch ein Verfahren gewonnen zu werden, umfassend die folgenden Schritte:
- Solubilisieren von mindestens 100 g/L Presskuchen von *Vaccinium macrocarpon* in Wasser,
- enzymatisches Hydrolysieren,
- Reinigen der Peptidfraktion.

11. Kosmetischer Wirkstoff nach einem der vorstehenden Ansprüche, **dadurch gekennzeichnet, dass** der Extrakt geeignet ist, um durch ein Extraktionsverfahren gewonnen zu werden, umfassend zwei enzymatische Hydrolysen von Presskuchen von *Vaccinium macrocarpon.*

12. Kosmetische Zusammensetzung für eine topische Anwendung, umfassend mindestens einen Wirkstoff nach einem der vorstehenden Ansprüche und ein physiologisch verträgliches Medium.

13. Kosmetische Zusammensetzung nach dem vorstehenden Anspruch, wobei der Wirkstoff mindestens zu 0,1 Gew.-% das Gesamtgewicht der Zusammensetzung darstellt.

14. Kosmetische Verwendung eines Wirkstoffs nach einem der Ansprüche 1 bis 11 oder einer kosmetischen Zusammensetzung nach einem der Ansprüche 12 oder 13 für einen Anti-Aging-Effekt.

15. Kosmetische Verwendung nach dem vorstehenden Anspruch für eine Anti-Falten-Wirkung und/oder zum Verbessern der Ausstrahlung des Teints und/oder Verbessern der Qualität der dermoepidermalen Verbindung und/oder Verbessern der Hauthomöostase.

## Claims

1. Cosmetic active substance comprising at least one extract of *Vaccinium macrocarpon,* **characterized in that** said extract comprises at least 80% peptides by weight of dry matter of the extract.

2. Cosmetic active substance according to the preceding claim, **characterized in that** said extract is obtained from the fruit of *Vaccinium macrocarpon.*

3. Cosmetic active substance according to one of the preceding claims, **characterized in that** said extract is obtained from *Vaccinium macrocarpon* presscakes.

4. Cosmetic active substance according to one of the preceding claims, **characterized in that** the extract is a hydrolysate of *Vaccinium macrocarpon.*

5. Cosmetic active substance according to the preceding claim, **characterized in that** the hydrolysate is an enzymatic hydrolysate.

6. Cosmetic active substance according to one of claims 4 or 5, **characterized in that** the hydrolysate comprises at least 80% peptides by weight of dry matter of the extract, said peptides having a size less than or equal to 2,000 Da.

7. Cosmetic active substance according to the preceding claim, **characterized in that** the size is between 243 and 2,000 Da.

8. Cosmetic active substance according to one of the preceding claims, **characterized in that** the peptides present in the hydrolysate comprise on average between 4 and 6 amino acids.

9. Cosmetic active substance according to the preceding claim, **characterized in that** said extract has a content of basic amino acid-rich cationic peptides greater than 20%.

10. Cosmetic active substance according to one of the preceding claims, **characterized in that** said extract is capable of being obtained by a method comprising the following steps:
- solubilizing at least 100 g/L of *Vaccinium Macrocarpon* presscakes in water,
- enzymatic hydrolysis,
- purifying the peptide fraction.

11. Cosmetic active substance according to one of the preceding claims, **characterized in that** said extract is capable of being obtained by an extraction method comprising two enzymatic hydrolyses of *Vaccinium macrocarpon* presscakes.

12. Cosmetic composition for topical application comprising at least one active substance according to one of the preceding claims and a physiologically acceptable medium.

13. Cosmetic composition according to the preceding claim, wherein the active substance represents at least 0.1 % by weight of the total weight of the composition.

14. Cosmetic use of an active substance according to one of claims 1 to 11 or a cosmetic composition according to one of claims 12 or 13, for an anti-aging effect.

15. Cosmetic use according to the preceding claim for an anti-wrinkle effect and/or for improving the radiance of the complexion and/or for improving the quality of the dermo-epidermal junction and/or for improving skin homeostasis.
